# EUROPÄISCHE PATENTSCHRIFT

(11) **EP 3 370 040 B1**
(45) Veröffentlichungstag und Bekanntmachung des Hinweises auf die Patenterteilung: **21.04.2021**
(21) Anmeldenummer: 17158911.2
(22) Anmeldetag: 02.03.2017
(51) Int. Cl.: G01F 22/00, A61J 1/18, A61M 5/36, B65B 3/00, A61J 1/22, G01F 25/00, A61M 5/178, G01F 11/02, G01N 35/10, B01L 3/02

(54) **VERFAHREN ZUR BESTIMMUNG EINES GASVOLUMENS EINER FERTIGSPRITZE UND VERWENDUNG DES VERFAHRENS ZUR EICHUNG EINER ANLAGE ZUM ABFÜLLEN EINER FERTIGSPRITZE**
METHOD OF DETERMINING THE GAS VOLUME OF A PRE-FILLED SYRINGE AND USE OF THE METHOD FOR CALIBRATING A MACHINE FOR FILLING SYRINGES
PROCÉDÉ DE DÉTERMINATION DU VOLUME DE GAZ DANS UNE SERINGUE PRÉREMPLIE ET USAGE DU PROCÉDÉ POUR CALIBRER UNE MACHINE DE REMPLISSAGE DE SERINGUES

(43) Veröffentlichungstag der Anmeldung: 05.09.2018
(73) Patentinhaber: Fresenius Kabi Austria GmbH, 8055 Graz (AT)
(72) Erfinder: HOHL, Karin, 8042 Graz (AT); HURDAX, Daniel, 8101 Gratkorn (AT)
(74) Vertreter: Fresenius Kabi Deutschland GmbH

(56) Entgegenhaltungen:
- US-A- 3 498 111
- US-A1- 2005 252 574
- US-A1- 2008 098 798
- US-A1- 2008 103 445
- US-A1- 2014 157 731
- US-A1- 2016 247 277
- SCHOENKNECHT T ET AL: "Prefilled syringes: Why new developments are important in injectable delivery today", INTERNET CITATION, 1. Januar 2005 (2005-01-01), Seiten 9-11, XP002608318, Gefunden im Internet: URL:http://www.ondrugdelivery.com/publicat ions/prefilled_syringes.pd [gefunden am 2010-11-02]

## Beschreibung

### Gebiet der Erfindung

Die Erfindung betrifft ein Verfahren zur Bestimmung des Gasvolumens einer Fertigspritze. Weiter betrifft die Erfindung die Verwendung des Verfahrens zur Eichung einer Anlage zum Abfüllen von Fertigspritzen.

### Hintergrund der Erfindung

Fertigspritzen sind sowohl mit als auch ohne Nadel bekannt. Es handelt sich dabei um Spritzen, die mit einer vorgegebenen Menge einer, vorzugsweise wirkstoffhaltigen, Flüssigkeit, beispielsweise eines Arzneimittels, befüllt sind, und welche vom Benutzer direkt verwendet werden können. Je nach Ausführungsform muss der Benutzer noch eine Kolbenstange anbringen und/oder eine Nadel aufsetzen und kann sodann die Spritze direkt verwenden.

Durch die Verwendung von vorbefüllten Fertigspritzen kann insbesondere eine sehr hohe Dosiergenauigkeit erreicht werden. In aller Regel befindet sich innerhalb der Fertigspritze ein kleines Gasvolumen, welches üblicherweise vor Verwendung durch Betätigung des Kolbens entfernt wird.

Relevant ist das Volumen des in der Fertigspritze vorhandenen Gasvolumens insbesondere bei der Einstellung und Eichung von Anlagen zur Befüllung der Fertigspritzen. Angestrebt wird sowohl ein möglichst gleiches Flüssigkeits- als auch ein möglich gleiches Gasvolumen. Die Messung des Gasvolumens ist allerdings nicht einfach.

Die Offenlegungsschrift WO 2009/099641 A2, welche eine Proteinzusammensetzung betrifft, beschreibt u.a. die Bestimmung des Gasraums in einer Spritze durch Messung der Gasblasengröße mittels eines Mikroskops oder einer Vergrößerungslinse. Dabei wird der Gasraum bzw. die Gasblase direkt in der vorbefüllten Spritze vermessen. Es sind Messungen von 0,1 mm bis 3,0 mm angegeben. Das Verfahren scheint zur Messung von größeren Gasblasen wenig geeignet zu sein. Weiter scheint es sich um eine berechnete Größe zu handeln, d.h. sphärische oder elliptische Gasblasen werden vermessen und das Volumen wird mathematisch bestimmt. Durch diese mathematische Berechnung entstehen Ungenauigkeiten, da die tatsächliche Form der Gasblase im Allgemeinen nicht dem mathematischen Modell entspricht. Das Verfahren ist zudem recht aufwändig.

US 2008/103445 A1, US 2005/252574 A1 und US 2008/098798 A1 offenbaren Verfahren zur Bestimmung eines durch Gas- oder Luftblasen gebildeten Gasvolumens in einer Fluidleitung mittels Gasblasendetektoren, wobei die Fluidleitungen insbesondere zur Verabreichung von Medikamenten dienen.

US 2014/157731 A1 und US 2016/247277 A1 offenbaren kameragestützte Verfahren zur Bestimmung eines Gasvolumens ("headspace") in Spritzen.

### Aufgabe der Erfindung

Der Erfindung liegt demgegenüber die Aufgabe zugrunde, ein Verfahren zur Bestimmung des Gasvolumens einer Fertigspritze bereit zu stellen, welches einfach durchführbar ist und eine hohe Genauigkeit bietet.

### Zusammenfassung der Erfindung

Die Aufgabe der Erfindung wird durch ein Verfahren zur Bestimmung des Gasvolumens in einer Fertigspritze, und durch die Verwendung des Verfahrens zur Eichung einer Anlage zum Abfüllen von Fertigspritzen gelöst.

Bevorzugte Ausführungsformen der Erfindung sind dem Gegenstand der abhängigen Ansprüche zu entnehmen. Die Erfindung betrifft zunächst ein Verfahren zur Bestimmung des Gasvolumens in einer Fertigspritze.

Unter einer Fertigspritze im Sinne der Erfindung wird ein als zumindest für eine Fertigspritze verwendbares Gehäuse verstanden, das mit einer, vorzugsweise wirkstoffhaltigen, Flüssigkeit, beispielsweise mit einem Arzneimittel, vorbefüllt ist und welches einen Kolben zum Austreiben der Flüssigkeit umfasst. Derartige Fertigspritzen können bereits eine Nadel umfassen. Eine Nadel kann aber auch aufgesetzt werden. Auch Die Kolbenstange zur Betätigung des Kolbens kann sowohl bereits am Kolben vormontiert sein als auch in abgetrennter Form vorliegen, wobei im letzteren Fall die Kolbenstange vor der Verwendung der Fertigspritze montiert werden muss. Eine Fertigspritze kann auch als vorgefüllte Spritze bezeichnet werden.

Gemäß dem erfindungsgemäßen Verfahren wird ein Röhrchen mit einer Volumenskala verwendet, welches an einem Ende, insbesondere an einem unteren Ende, mit einem Septum verschlossen ist.

Das Septum ist derart ausgebildet, dass es mit einer Nadel eingestochen werden kann.

Zunächst wird das Röhrchen mit einer Flüssigkeit, insbesondere mit einer eingefärbten Flüssigkeit, befüllt.

Sodann wird das Septum mit der Fertigspritze eingestochen und ein in der Fertigspritze enthaltenes Gasvolumen in das Röhrchen transferiert, wobei das Gasvolumen zwischen zumindest zwei Flüssigkeitsphasen in dem Röhrchen eingeschlossen wird.

Das Transferieren des Gasvolumens erfolgt insbesondere, indem zunächst die Nadel der Fertigspritze mit Flüssigkeit befüllt wird. Dazu wird die Spritze mit der Nadel nach unten solange betätigt, bis Flüssigkeit anfängt, aus der Nadel auszutreten. Sodann wird das Gasvolumen bzw. die Gasblase an ein vorderes Ende des Innenvolumens der Spritze gebracht, indem die Spritze in eine Position mit der Nadel nach oben verbracht wird und die Gasblase, gegebenenfalls unter Klopfen, an das obere Ende ansteigt.

Sodann wird das Septum mit der Nadel eingestochen und es wird die Spritze betätigt. Dabei tritt zuerst die Flüssigkeit aus der vorab befüllten Nadel aus. Anschließend wird die Gasblase in das Röhrchen transferiert. Nach dem Transferieren wird noch weitere Flüssigkeit in das Röhrchen transferiert, vorzugsweise bis die Gasblase an einer Position ist, an welcher sie gut ablesbar ist.

Es kann dabei auch vorkommen, dass sich die Gasblase in zwei oder mehr Gasvolumina aufteilt, welche innerhalb des Röhrchens jeweils durch Flüssigkeit voneinander beabstandet sind. In so einem Fall sind die Gasvolumina zu addieren, um das in der Fertigspritze vorhandene gesamte Gasvolumen zu bestimmen.

Durch die Erfindung ist es möglich, auf einfache Weise unmittelbar das in der Fertigspritze vorhandene Gasvolumen mit hoher Genauigkeit zu bestimmen.

Bei einer bevorzugten Ausführungsform der Erfindung wird ein Septum mit einer Führung für eine Nadel verwendet.

Auf diese Weise ist es möglich, die Nadelspitze exakt zum Röhrchen zu führen, welches vorzugsweise als Glasröhrchen ausgebildet ist.

Es wird insbesondere ein auf "In" geeichtes Röhrchen verwendet, also ein Röhrchen, bei welchem das Röhrchen auf das in das Röhrchen eingebrachte Volumen geeicht ist. Die Gasblasengröße kann so unmittelbar abgelesen werden.

Vorzugsweise wird ein Röhrchen mit einer ml- oder µl-Skala verwendet. Vorzugsweise ist die Volumenskala direkt an dem Röhrchen angebracht.

Durch die Erfindung können insbesondere Gasvolumina von 1 - 200 µl, besonders bevorzugt von 5 - 80 µl, gemessen werden.

Vorzugsweise wird das Röhrchen mit einer Flüssigkeit gefüllt, die mit der Flüssigkeit, mit der die Fertigspritze befüllt ist, mischbar ist.

Bei Fertigspritzen, die eine wässrige Lösung enthalten, kann beispielsweise Wasser, insbesondere eingefärbtes Wasser, zum Befüllen der Spritze und/oder des Röhrchens verwendet werden.

Für ölbasierte Lösungen können Öle, beispielsweise Sojaöl oder Sesamöl, verwendet werden. Weiter können Alkohole, insbesondere Alkanole, verwendet werden.

Durch die Verwendung einer Flüssigkeit zum Vorbefüllen, die mit der Flüssigkeit, mit der die Fertigspritze gefüllt ist, mischbar ist, wird insbesondere vermieden, dass sich Ablagerungen im Röhrchen bilden.

Bei einer weiteren Ausführungsform der Erfindung wird bzw. ist ein oberes Ende des Röhrchens mit einem Absperrventil verbunden, insbesondere über einen Schlauch. Das Absperrventil wird nach dem Transferieren des in der Fertigspritze vorhandenen Gasvolumens geschlossen. Sodann kann die Fertigspritze abgenommen und das Gasvolumen auf der Volumenskala abgelesen werden. Durch das Absperrventil wird sichergestellt, dass die Flüssigkeit nicht beim Abziehen der Spritze zurückläuft, was zu einer Verschiebung des Gasvolumens, welches nun im Röhrchen eingeschlossen ist, führen würde.

Das erfindungsgemäße Verfahren wird insbesondere verwendet, um Anlagen zum Abfüllen von Fertigspritzen zu eichen. In einer Ausführungsform ist das Verfahren dadurch gekennzeichnet, dass es zur Eichung einer Anlage zum Abfüllen von Fertigspritzen verwendet wird und dass Fertigspritzen mit der geeichten Anlage abgefüllt werden.

Durch die Erfindung ist es möglich, eine Charge von Fertigspritzen herzustellen, bei welcher das Gasvolumen eine geringe Standardabweichung hat, insbesondere von Fertigspritzen mit jeweils einem Gasvolumen, wobei die Standardabweichung des Gasvolumens innerhalb der Charge weniger als 3 µl, bevorzugt weniger als 1 µl, beträgt.

Die Erfindung betrifft nicht die Messvorrichtung, welche für das zuvor beschriebene Verfahren verwendet wird.

Die Messvorrichtung umfasst ein Röhrchen mit einer Volumenskala, wobei das Röhrchen an einem unteren Ende mit einem durchstoßbaren Septum verschlossen ist. Es handelt sich insbesondere um ein Glasröhrchen.

Vorzugsweise ist das Röhrchen spannungsfrei zwischen zwei Armen der Messvorrichtung eingespannt. So können auch dünne Glasröhrchen, insbesondere solche, die eine Skala mit einer Ablesegenauigkeit im µl-Bereich aufweisen, verwendet werden, ohne dass die Gefahr einer Zerstörung besteht.

Bei einer bevorzugten Ausführungsform ist das Röhrchen an einem unteren Ende mit dem Septum eingespannt und an einem oberen Ende mit einer elastischen Hülse.

Insbesondere werden ein Septum und/oder eine Hülse aus einem Silikon verwendet. Es hat sich gezeigt, dass Silikone auch nach vielfachem Durchstechen immer noch ein dichtes Septum bilden.

Um das Röhrchen einspannen zu können, umfasst das Septum vorzugsweise ein Sackloch zur Aufnahme des Röhrchens. Der Durchmesser des Sacklochs ist vorzugsweise derart bemessen, dass das Röhrchen im Sackloch eingeklemmt wird.

Bei einer bevorzugten Ausführungsform der Erfindung umfasst das Septum einen Einsatz mit einer Nadelführung. Es handelt sich dabei insbesondere um einen Einsatz aus Metall oder Kunststoff, welcher in dem Septum, insbesondere in einem Silikonseptum, eingeschlossen ist. Der Einsatz weist einen dünnen, an die Nadel angepassten Durchlass auf, so dass die Nadel beim Einstechen des Septums genau über dem unteren Ende des Röhrchens positioniert ist.

Die Nadelführung kann an ihrem unteren Ende zum besseren Einführen auch einen Konus umfassen.

Die Nadel kann so auf einfache Weise in das Röhrchen eingeführt werden, wobei das Septum nach dem Abnehmen der Spritze als Dichtung dient.

Die Messvorrichtung umfasst eine Spannvorrichtung zum Festklemmen an einem Stativ. So kann auf einfache Weise die Messvorrichtung fest und sicher positioniert angebracht werden. Die Spannvorrichtung kann insbesondere eine Klemmleiste umfassen, die mittels Schrauben festgezogen wird.

Da vorzugsweise auch das Röhrchen eingeklemmt ist, kann über eine separate Klemmeinrichtung des Röhrchens dieses auch auf einfache Weise ausgetauscht werden, um den Messbereich anzupassen.

### Kurzbeschreibung der Zeichnungen

Die Erfindung soll im Folgenden, Bezug nehmend auf ein Ausführungsbeispiel, anhand der Zeichnungen Fig. 1 bis Fig. 7 näher erläutert werden.
Fig. 1 ist eine schematische Ansicht, anhand welcher erläutert werden soll, wie eine Messvorrichtung zur Bestimmung des Gasvolumens einer Fertigspritze verwendet wird.
Fig. 2 ist eine perspektivische Detaildarstellung der Messvorrichtung.
Fig. 3 ist eine perspektivische Ansicht des verwendeten Septums.
Fig. 4 ist ein Schnitt des in Fig. 3 dargestellten Septums.

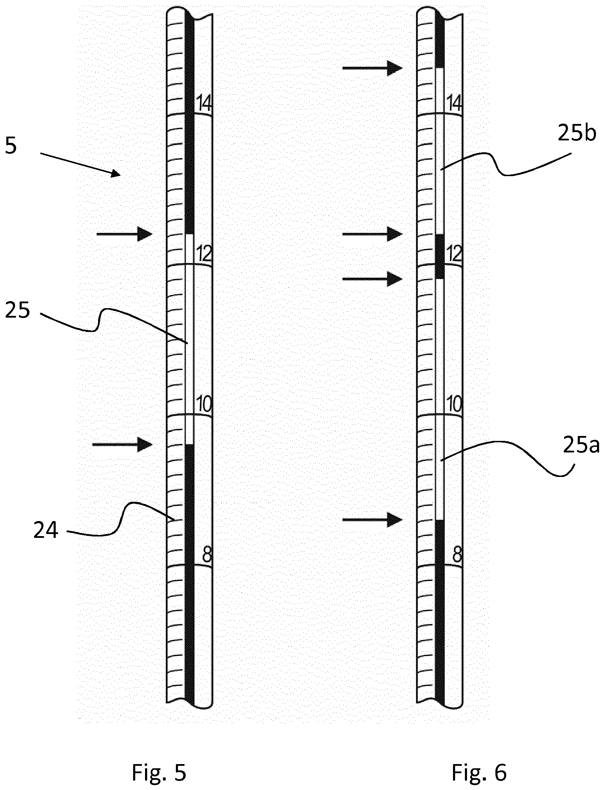

Anhand von Fig. 5 und Fig. 6 soll, Bezug nehmend auf eine Detaildarstellung des Röhrchens, das Ablesen des Gasvolumens erläutert werden.

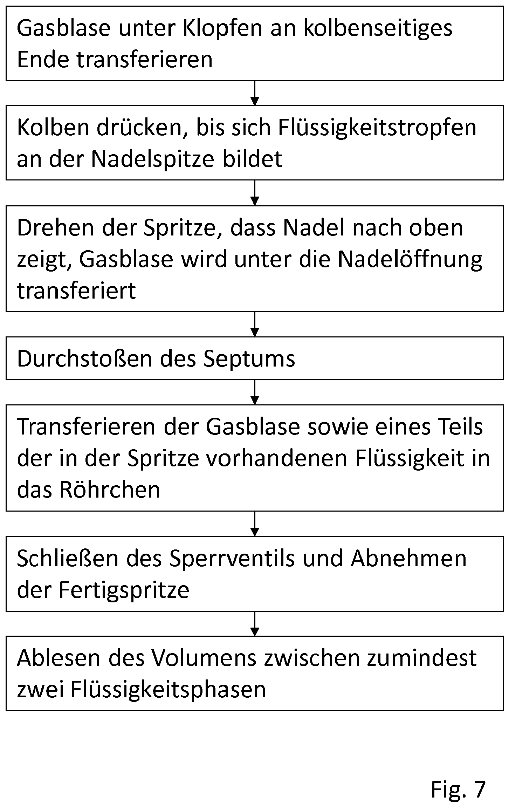

Fig. 7 ist ein Flussdiagramm, in welchem die Schritte des erfindungsgemäßen Verfahrens gemäß einer Ausführungsform erläutert werden.

### Detaillierte Beschreibung der Zeichnungen

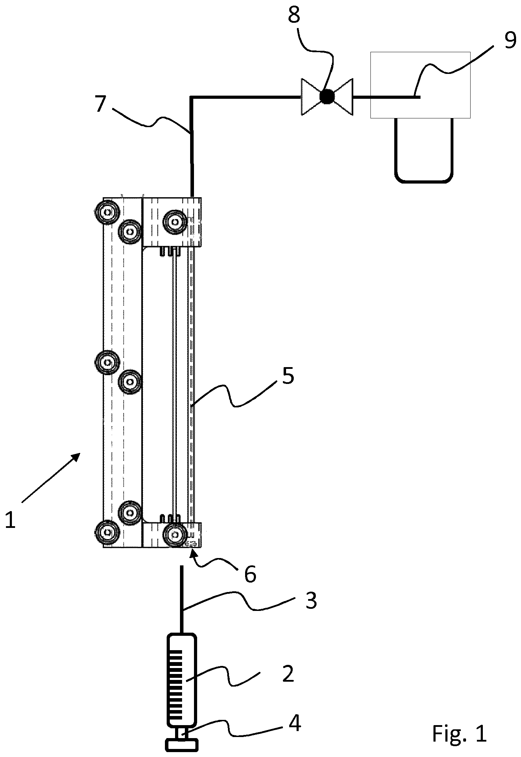

Fig. 1 ist eine schematische Ansicht, in welcher eine Messvorrichtung 1 dargestellt ist, die ein Röhrchen 5 mit einer Volumenskala 24 (siehe dazu Fig. 5 und 6) umfasst.

Das Röhrchen 5 ist vorzugsweise als Glasröhrchen, insbesondere mit einem Innendurchmesser zwischen 0,1 und 1 mm, bevorzugt zwischen 0,15 und 0,4 mm, ausgebildet. Vorzugsweise besitzt das Röhrchen eine Länge von 10 cm bis 50 cm, bevorzugt von 20 cm bis 40 cm.

An einem unteren Ende des in der Messvorrichtung 1 eingespannten Röhrchens 5 befindet sich ein Septum 6, welches mit der Nadel 3 einer Fertigspritze 2 durchstoßen werden kann.

Das obere Ende des Röhrchens 5 ist mit einem Schlauch 7 verbunden, der zu einem Absperrventil 8 führt. Hinter dem Absperrventil 8 befindet sich ein Überlauf 9, über welchen Flüssigkeit ausfließen und in einem Behältnis aufgefangen werden kann, von wo aus die Flüssigkeit sodann verworfen werden kann.

Zur Messung der Größe des Gasvolumens in der Fertigspritze 2 wird das Gasvolumen durch Betätigen der Kolbenstange 4 in das Röhrchen 5 transferiert, indem zunächst die Fertigspritze 2 mit der Nadel 3 in das Septum 6 gestoßen wird und sodann die Kolbenstange 4 betätigt wird.

Dabei tritt zunächst die in der Nadel 3 vorhandene Flüssigkeit in das Röhrchen 5 ein, sodann folgt die Gasblase. Der Benutzer betätigt die Kolbenstange 4 so weit, bis weitere Flüssigkeit aus der Fertigspritze 2 nachströmt und das nunmehr in dem Röhrchen 5 vorhandene Gasvolumen gut abgelesen werden kann.

Anschließend schließt der Benutzer das Absperrventil 8 und nimmt die Fertigspritze 2 ab, um sodann unmittelbar das Volumen ablesen zu können.

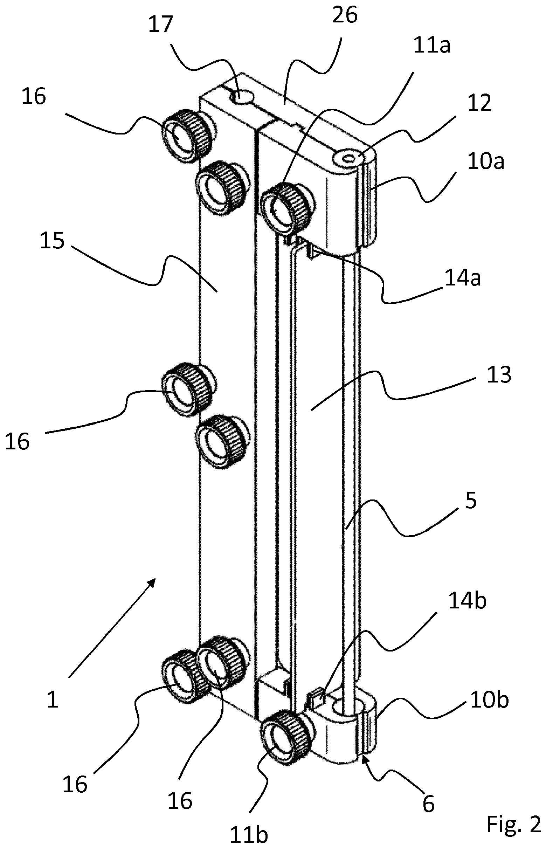

Fig. 2 ist eine perspektivische Detaildarstellung der in Fig. 1 dargestellten Messvorrichtung 1. Die Messvorrichtung 1 umfasst ein Gehäuse mit zwei Armen 10a, 10b. Über die Arme 10a und 10b wird das Röhrchen 5 spannungsfrei eingespannt.

Hierzu haben die Arme 10a, 10b jeweils eine Schraube 11a, 11b, welche vorzugsweise als Rädelschraube oder Flügelschraube ausgebildet ist, und demzufolge werkzeuglos betätigt werden kann.

Das Röhrchen 5 ist sowohl an einem oberen Arm 10a als auch an einem unteren Arm 10b mit einem das Röhrchen 5 umgebenden Elastomerelement eingeklemmt. An dem oberen Ende ist das Röhrchen 5 von einer Hülse 12 aus einem Elastomermaterial, insbesondere einer Silikonhülse, umgeben. An dem unteren Ende ist das Röhrchen 5 vom Septum 6 umgeben und mit dem Septum 6 eingespannt. Das Septum 6 wird aus einem Elastomermaterial gebildet. Unter einem Elastomermaterial im Sinne der Erfindung werden alle Arten von elastischen Kunststoffmaterialien verstanden, z.B. auch elastische Thermoplaste.

Weiter tragen in diesem Ausführungsbeispiel die Arme 10a, 10b eine Kontrastscheibe 13, welche in den Halterungen 14a und 14b eingeklemmt ist. Die Kontrastscheibe 13 dient dem besseren Ablesen der Volumenskala 24 des Röhrchens 5. Je nach Farbe der in das Röhrchen 5 eingebrachten Flüssigkeit kann die Kontrastscheibe 13 durch eine Kontrastscheibe 13 einer anderen Farbe einfach ausgetauscht werden.

Weiter umfasst die Messvorrichtung 1 eine Spannvorrichtung 15, welche in diesem Ausführungsbeispiel aus einer auf eine Grundplatte 26 der Messvorrichtung 1 aufgebrachten Klemmplatte besteht.

Zwischen der Grundplatte 26 und der restlichen Spannvorrichtung 15 befindet sich ein Durchgangsloch 17, welches der Aufnahme der Stange eines Stativs dient (nicht dargestellt).

Über die Schrauben 16, welche vorzugsweise ebenfalls als Rädel- oder Flügelschrauben ausgebildet sind, kann die Messvorrichtung 1 an einem Stativ sicher festgeklemmt werden.

Die Grundplatte 26 ist vorzugsweise einstückig mit einem hinteren Teil der Arme 10a, 10b ausgebildet. So ist eine spannungsfreie Aufnahme des Röhrchens 5 zwischen den Armen 10a und 10b sichergestellt.

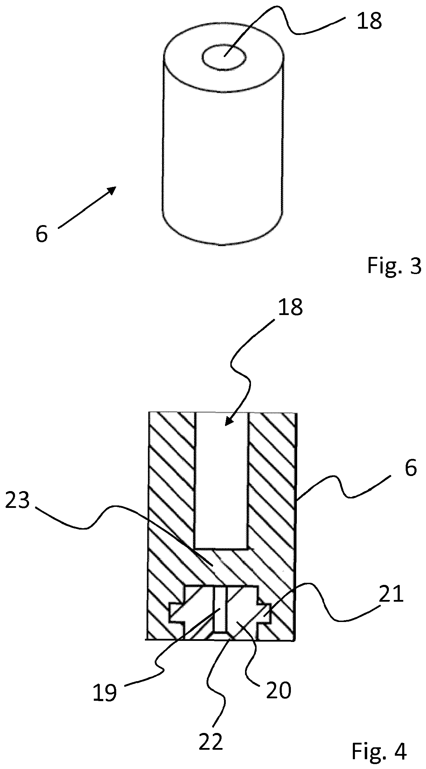

Fig. 3 ist eine perspektivische Detaildarstellung des Septums 6.

Das Septum 6 ist, insbesondere um dieses zusammen mit dem Röhrchen 5 einspannen zu können, in diesem Ausführungsbeispiel kreiszylindrisch ausgebildet.

Auf seiner Oberseite umfasst das Septum 6 ein Sackloch 18, welches der Aufnahme des Röhrchens 5 dient. So dient das Septum 6 nicht nur dem Einführen der Nadel 3, sondern ist gleichzeitig als das Röhrchen 5 umschließendes Elastomerelement zum Einspannen des Röhrchens ausgebildet.

Fig. 4 ist ein Schnitt des in Fig. 3 dargestellten Septums 6.

Das Septum 6 umfasst einen unteren Bereich, in welchem eine Nadelführung 19 eingebracht ist.

In diesem Ausführungsbeispiel ist zum Ausbilden der Nadelführung 19 ein Einsatz 20 in das aus Silikon bestehende Septum 6 eingebracht, vorzugsweise eingegossen.

Der Einsatz 20 besteht aus einem härteren Material als das restliche Septum 6. Insbesondere ist der Einsatz 20 aus einem Metall und/oder aus einem Kunststoff gefertigt.

Der Einsatz 20 wird aufgrund des Kragens 21 formschlüssig in dem Septum 6 gehalten.

An seinem unteren Ende umfasst der Einsatz 20 einen Konus 22, um das Ende der Nadel 3 leichter in die Nadelführung 19 einführen zu können.

Wird die Nadel 3 eingeführt, ist diese mittig unter dem Sackloch 18 und damit genau unter dem unteren Ende des Röhrchens 5 positioniert.

Nach Durchstoßen des Durchstoßbereichs 23, welcher von der Nadelführung 19 bis zum unteren Ende des Röhrchens 5 führt, kann Flüssigkeit sowie das Gasvolumen von der Fertigspritze 2 in das Röhrchen 5 transferiert werden.

Zumindest der Durchstoßbereich 23 besteht vorzugsweise aus einem Elastomermaterial mit einer Härte Shore A (bei RT) zwischen 20 und 70, besonders bevorzugt zwischen 20 und 50. Der Durchstoßbereich verschließt sich nach dem Herausziehen der Nadel 3 selbstständig. Vorzugsweise ist der Durchstoßbereich zwischen 1 und 6 mm, besonders bevorzugt zwischen 3 und 5 mm, dick.

Wie in der Detailansicht des Röhrchens 5 in Fig. 5 dargestellt, ist das Gasvolumen 25 nach dem Transferieren in das Röhrchen 5 zwischen Flüssigkeit eingeschlossen.

Durch Differenzbildung des Volumens des mit einem Pfeil markierten oberen Endes der Volumenskala 24 mit dem unteren Ende der Volumenskala 24 kann auf einfache Weise das Volumen bestimmt werden. In diesem Ausführungsbeispiel ist die Volumenskala 24 auf Zehntel ml geeicht.

In der Darstellung gemäß Fig. 6 hat sich das in der Fertigspritze 2 vorhandene Gasvolumen in zwei Gasvolumina 25a, 25b aufgeteilt, welche zusammengerechnet werden müssen. Hierzu werden durch Differenzbildung die Größen der Gasvolumina 25a, 25b bestimmt und die Volumina werden addiert.

Fig. 7 zeigt ein Flussdiagramm eines Ausführungsbeispiels der Erfindung mit den folgenden Verfahrensschritten:
Zur Bestimmung des Gasvolumens 25, 25a, 25b einer Fertigspritze 2 wird zunächst die in der Fertigspritze 2 vorhandene Gasblase, gegebenenfalls unter leichtem Klopfen, an ein kolbenseitiges Ende transferiert. Die Spitze der Spritze 2 zeigt dabei nach unten.

Sofern es sich um eine Fertigspritze 2 ohne Nadel 3 handelt, wird vor dem Klopfen zunächst eine Nadel 3 aufgesetzt.

Der Kolben der Fertigspritze 2 wird sodann gedrückt, bis sich ein Flüssigkeitstropfen an der Nadelspitze bildet. Die Nadel 3 ist nunmehr mit Flüssigkeit gefüllt.

Sodann wird die Fertigspritze 2 so gedreht, dass die Nadel 3 nach oben zeigt. Die Gasblase wird, gegebenenfalls wieder unter leichtem Klopfen, unter die Nadelöffnung transferiert, indem die Gasblase in der Flüssigkeit aufsteigt.

Anschließend wird das Septum 6 der Messvorrichtung 1 mit der Nadel 3 durchstoßen. Durch möglichst gleichmäßigen Druck auf den Kolben der Fertigspritze 2 wird die vollständige Gasblase sowie ein Teil der in der Fertigspritze 2 vorhandenen Flüssigkeit in das Röhrchen 5 transferiert.

In dem Röhrchen 5 mit einer Volumenskala ist nunmehr die eingeschlossene Gasblase, also das zu bestimmende Gasvolumen 25, 25a, 25b, gut erkennbar.

Zum Ablesen wird vorzugsweise das Absperrventil 8 geschlossen und erst dann die Fertigspritze 2 abgenommen.

Sodann wird auf der Volumenskala ein Volumen vom Beginn der Gasblase 25, 25a, 25b bis zum Ende der Gasblase 25, 25a, 25b abgelesen. Durch Differenzbildung kann auf einfache Weise die absolute Größe der Gasblase 25, 25a, 25b bestimmt werden.

Falls die Gasblasen 25a, 25b durch Einschlüsse von Flüssigkeit unterteilt sind, werden diese einzeln abgelesen und die Volumina der einzelnen Gasblasen 25a, 25b addiert zum Gesamtgasvolumen.

Zwischen den einzelnen Messungen kann das Röhrchen 5 wieder mit Flüssigkeit befüllt werden. Falls ausreichend Produkt in der Fertigspritze vorhanden ist, kann dieser Schritt aber auch entfallen.

In der Volumenskala sind vorzugsweise Volumina von über 2 µl mit einer Genauigkeit von mindestens 1 µl ablesbar und bestimmbar. Es versteht sich, dass eine untere Grenze des Messverfahrens, insbesondere eine untere Grenze von unter 2 µl dadurch gebildet werden kann, dass kleinere Gasblasen durch Adhäsion an einer Wand der Fertigspritze 2 hängen bleiben können.

Durch die Erfindung wird ein sehr einfaches und zuverlässiges Verfahren bereitgestellt, mit der das in einer Fertigspritze 2 vorhandene Gasvolumen zerstörungsfrei und mit hoher Genauigkeit gemessen werden kann.

### Bezugszeichenliste

1 Messvorrichtung
2 Fertigspritze
3 Nadel
4 Kolbenstange
5 Röhrchen
6 Septum
7 Schlauch
8 Absperrventil
9 Überlauf
10a, 10b Arm
11a, 11b Schraube
12 Hülse
13 Kontrastscheibe
14a, 14b Halterung
15 Spannvorrichtung
16 Schraube
17 Durchgangsloch
18 Sackloch
19 Nadelführung
20 Einsatz
21 Kragen
22 Konus
23 Durchstoßbereich
24 Volumenskala
25, 25a, 25b Gasvolumen
26 Grundplatte

## Patentansprüche

1. Verfahren zur Bestimmung eines Gasvolumens einer Fertigspritze (2) umfassend die Schritte:
- Bereitstellen eines Röhrchens (5) mit einer Volumenskala (24), welches an einem Ende mit einem Septum (6) verschlossen ist,
- Füllen des Röhrchens (5) mit einer Flüssigkeit,
- Einstechen des Septums (6) mit der Fertigspritze (2),
- Transferien des in der Fertigspritze (2) enthaltenen Gasvolumens in das Röhrchen (5), wobei das Gasvolumen zwischen zumindest zwei Phasen von Flüssigkeit eingeschlossen wird,
- Ablesen des Gasvolumens auf der Volumenskala (24).

2. Verfahren nach dem vorstehenden Anspruch, **dadurch gekennzeichnet, dass** ein Septum (6) mit einer Führung (19) für eine Nadel (3) der Fertigspritze (2) verwendet wird.

3. Verfahren nach einem der vorstehenden Ansprüche, **dadurch gekennzeichnet, dass** ein auf "In" geeichtes Röhrchen (5), insbesondere mit einer ml- oder µl-Skala, verwendet wird.

4. Verfahren nach einem der vorstehenden Ansprüche, **dadurch gekennzeichnet, dass** ein Gasvolumen von 1 bis 200 µl, vorzugsweise von 5 bis 80 µl, gemessen wird.

5. Verfahren nach einem der vorstehenden Ansprüche, **dadurch gekennzeichnet, dass** das Röhrchen mit einer Flüssigkeit gefüllt wird, die mit der Flüssigkeit, mit der die Fertigspritze (2) befüllt ist, mischbar ist.

6. Verfahren nach einem der vorstehenden Ansprüche, **dadurch gekennzeichnet, dass** ein oberes Ende des Röhrchens (5) mit einem Absperrventil (8) verbunden wird, wobei das Absperrventil (8) nach dem Transferieren des in der Fertigspritze (2) vorhandenen Gasvolumens geschlossen wird, die Fertigspitze sodann abgenommen und das Gasvolumen auf der Volumenskala (24) abgelesen wird.

7. Verfahren nach einem der vorstehenden Ansprüche, **dadurch gekennzeichnet, dass** es zur Eichung einer Anlage zum Abfüllen von Fertigspritzen (2) verwendet wird und dass Fertigspritzen mit der geeichten Anlage abgefüllt werden.

8. Verwendung eines Verfahrens nach einem der vorstehenden Ansprüche zur Eichung einer Anlage zum Abfüllen von Fertigspritzen (2).

9. Verwendung nach dem vorstehenden Anspruch, **dadurch gekennzeichnet, dass** mit der geeichten Anlage eine Charge von Fertigspritzen (2) abgefüllt wird.

10. Verwendung nach dem vorstehenden Anspruch, **dadurch gekennzeichnet, dass** zumindest acht Fertigspritzen mit einem Gasvolumen abgefüllt werden, wobei die Standardabweichung des Gasvolumens innerhalb der Charge weniger als 3 µl, bevorzugt weniger als 1 µl beträgt.

11. Verfahren nach dem vorstehenden Anspruch 1, **dadurch gekennzeichnet, dass** das Röhrchen (5) spannungsfrei zwischen zwei Armen (10a, 10b) eingespannt wird.

12. Verfahren nach Anspruch 2, **dadurch gekennzeichnet, dass** das Septum (6) aus einem Silikon besteht.

13. Verfahren nach einem der vorstehenden Ansprüche 2 oder 12, **dadurch gekennzeichnet, dass** das Septum einen Einsatz (20) mit einer Nadelführung (19) umfasst und/oder dass das Septum (6) ein Sackloch (18) zur Aufnahme des Röhrchens (5) umfasst.

14. Verfahren nach Anspruch 1, **dadurch gekennzeichnet, dass** eine Spannvorrichtung (15) zum Festklemmen des Röhrchens (5) an einem Stativ bereitgestellt wird.

## Claims

1. Method for determining a gas volume of a pre-filled syringe (2) which comprises the steps of:
- providing a small tube (5) with a volume scale (24) which is closed at one end with a septum (6),
- filling the small tube (5) with a liquid,
- perforating the septum (6) with the pre-filled syringe (2),
- transferring the gas volume contained in the pre-filled syringe (2) to the small tube (5), wherein the gas volume is enclosed between at least two liquid phases,
- reading the gas volume in the volume scale (24).

2. Method according to the preceding claim, **characterized in that** a septum (6) with a guide (19) for a needle (3) of the pre-filled syringe (2) is used.

3. Method according to one of the preceding claims, **characterized in that** a small tube (5) calibrated in "In", particularly with a ml or µl scale, is used.

4. Method according to one of the preceding claims, **characterized in that** a gas volume of 1 to 200 µl, preferably 5 to 80 µl, is measured.

5. Method according to one of the preceding claims, **characterized in that** the small tube is filled with a liquid which can be mixed with the liquid with which the pre-filled syringe (2) is filled.

6. Method according to one of the preceding claims, **characterized in that** an upper end of the small tube (5) is connected with a separating valve (8), wherein the separating valve (8) is closed after transferring the gas volume existing in the pre-filled syringe (2), then the prefilled syringe is extracted and the gas volume is read on the volume scale (24).

7. Method according to one of the preceding claims, **characterized in that** it is used to calibrate a piece of equipment for filling pre-filled syringes (2) and **in that** pre-filled syringes are filled using the calibrated equipment.

8. Use of a method according to one of the preceding claims for calibrating a piece of equipment for filling pre-filled syringes (2).

9. Use according to the preceding claim, **characterized in that** a batch of pre-filled syringes (2) is filled with the calibrated equipment.

10. Use according to the preceding claim, **characterized in that** at least eight pre-filled syringes are filled with a gas volume, wherein the standard deviation of the gas volume in the batch is less than 3 µl, preferably less than 1 µl.

11. Method according to the preceding claim 1, **characterized in that** the small tube (5) is held without stress between two arms (10a, 10b).

12. Method according to claim 2, **characterized in that** the septum (6) is comprised of a silicone.

13. Method according to one of the preceding claims 2 or 12, **characterized in that** the septum comprises an insert (20) with a needle guide (19), and/or **in that** the septum (6) comprises a blind hole (18) for housing the small tube (5).

14. Method according to claim 1, **characterized in that** a holding device (15) for immobilizing the small tube (5) in a support is provided.

## Revendications

1. Procédé de détermination du volume de gaz dans une seringue (2) préremplie comprenant les étapes de:
- fournir un tube (5) ayant une échelle (24) de volume qui est fermé à une extrémité par une cloison (6),
- remplir le tube (5) avec un liquide,
- perforer la cloison (6) avec la seringue (2) préremplie,
- transférer le volume de gaz contenu dans la seringue (2) préremplie au tube (5), dans lequel le volume de gaz est enfermé entre au moins deux phases de liquide,
- lire le volume de gaz sur l'échelle (24) de volume.

2. Procédé selon la revendication précédente, **caractérisé en ce qu'**il utilise une cloison (6) ayant un guide (19) pour une aiguille (3) de la seringue (2) préremplie.

3. Procédé selon l'une des revendications précédentes, **caractérisé en ce que** l'on utilise un tube (5) calibré en "In", en particulier avec une échelle de ml ou µl.

4. Procédé selon l'une des revendications précédentes, **caractérisé en ce que** l'on mesure un volume de gaz allant de 1 à 200 µl, de préférence de 5 à 80 µl.

5. Procédé selon l'une des revendications précédentes, **caractérisé en ce que** le tube est rempli d'un liquide qui peut se mélanger avec le liquide remplissant la seringue (2) préremplie.

6. Procédé selon l'une des revendications précédentes, **caractérisé en ce qu'**une extrémité supérieure du tube (5) es reliée à une soupape (8) de séparation, dans lequel la soupape (8) de séparation est fermée après avoir transféré le volume de gaz existant dans la seringue (2) préremplie, ensuite la seringue préremplie est extraite et le volume de gaz est lu sur l'échelle (24) de volume.

7. Procédé selon l'une des revendications précédentes, **caractérisé en ce qu'**il est utilisé pour calibrer une machine de remplissage des seringues (2) préremplies et **en ce que** les seringues préremplies sont remplies avec la machine calibrée.

8. Usage d'un procédé selon l'une des revendications précédentes pour calibrer une machine de remplissage des seringues (2) préremplies.

9. Usage selon la revendication précédente, **caractérisé en ce qu'**avec la machine calibrée on remplie un lot de seringues (2) préremplies.

10. Usage selon la revendication précédente, **caractérisé en ce qu'**au moins huit seringues préremplies sont remplies d'un volume de gaz, dans lequel l'écart typique du volume de gaz dans le lot est inférieur à 3 µl, de préférence inférieur à 1 µl.

11. Procédé selon la revendication précédente 1, **caractérisé en ce que** le tube (5) est maintenu sans tensions entre deux bras (10a, 10b).

12. Procédé selon la revendication 2, **caractérisé en ce que** la cloison (6) est composée d'une silicone.

13. Procédé selon l'une des revendications précédentes 2 ou 12, **caractérisé en ce que** la cloison comprend un insert (20) avec un guide (19) d'aiguille et/ou **en ce que** la cloison (6) comprend un orifice (18) borgne pour loger le tube (5).

14. Procédé selon la revendication 1, **caractérisé en ce que** l'on fournit un dispositif (15) de maintien pour immobiliser le tube (5) dans un support.
